# EUROPEAN PATENT APPLICATION

(11) **EP 1 479 766 A1**
(43) Date of publication of application: **24.11.2004**
(21) Application number: 03708505.7
(22) Date of filing: 06.03.2003
(51) Int. Cl.: C12N 15/00, C07H 21/04, B82B 1/00, B82B 3/00, A61K 9/51

(54) **DNA NANOCAGE BY SELF-ORGANIZATION OF DNA AND PROCESS FOR PRODUCING THE SAME, AND DNA NANOTUBE AND MOLECULE CARRIER USING THE SAME**

(30) Priority: 07.03.2002 JP 2002061504
(71) Applicant: KYUSHU TLO COMPANY, LIMITED, Fukuoka-shi, Fukuoka 812-0053 (JP)
(72) Inventor: MATSUURA, Kazunori, Fukuoka-shi, Fukuoka 813-0043 (JP); KIMIZUKA, Nobuo, Fukuoka-shi, Fukuoka 813-0016 (JP); YAMASHITA, Taro, Fukuoka-shi, Fukuoka 813-0062 (JP)
(74) Representative: Paget, Hugh Charles Edward
(86) International application number: PCT/JP2003/002644
(87) International publication number: WO 2003/074693

(57) **Abstract**

The invention provides a process for producing DNA nanocages, characterized by comprising a step of two-dimensionally assembling three types of oligonucleotides by hybridization to form tridirectionally branched double-strand DNA having self-complementary chains in the terminals, and a step of three-dimensionally self-organizing the resulting tridirectionally branched double-strand DNAs so as to consume all of the self-complementary terminals. Since the DNA nanocages according to the invention can easily be formed from DNAs by one-step procedure and include nanoparticles in the interior, the DNA nanocages are significantly effective for the development of functional materials using DNAs.

SEQUENCE LISTING

<110> Kyushu TLO Company, Limited

<120> DNA Nanocage by Self-Organization of DNA and Process for
Producing the Same, and DNA Nanotube and Molecule Carrier
Using the Same

<130> WJW/FP6245989

<140> EP 03708505.7
<141> 2003-03-06

<150> PCT/JP03/02644
<151> 2003-03-06

<150> JP 2002-061504
<151> 2002-03-07

<160> 3

<170> PatentIn version 3.1

<210> 1
<211> 30
<212> DNA
<213> artificial

<220>
<223> oligonucleotide synthesized by phosphoramidite solid-synthetic
method using DNA automatic synthesizer

<400> 1
ggcgtggtag accgcactcg aaaaattttt 30

<210> 2
<211> 30
<212> DNA
<213> artificial

<220>
<223>  oligonucleotide synthesized by phosphoramidite solid-synthetic
       method using DNA automatic synthesizer

<400>  2
cgagtgcggt gacgatgcct aaaaattttt                                        30

<210>  3
<211>  30
<212>  DNA
<213>  artificial

<220>
<223>  oligonucleotide synthesized by phosphoramidite solid-synthetic
       method using DNA automatic synthesizer

<400>  3
aggcatcgtc ctaccacgcc aaaaattttt                                        30

## Description

### Technical Field

The present invention relates to DNA nanocages which are novel molecular assemblies by self-organization of sequence-designed, tridirectionally branched double-strand DNAs and a process for producing the same, and DNA nanotube, and molecular carrier using the same.

### Background Art

In recent years, molecular machines using self-organization of DNAs have been actively developed in the field of nanotechnology.

Information of a DNA is encoded in the primary sequence of nucleotides by its base units: adenine (A), guanine (G), cytosine (C) and thymine (T). Single strand of DNA has unique character that it recognizes the complementary chain and is bound thereto by hybridization to form a double-strand nucleic acid. This can take place by formation of base pairs inherent in nucleic acid such that A recognizes T and G recognizes C. A given sequence is hybridized to an adequate and complementary sequence alone. Accordingly, high base sequence specificity is provided at atomic and molecular levels. Thus, DNA is quite interesting in not only its functionality but also the structural morphology, and studies have been made in bioinformatics as "human genome project" or the like.

N. C. Seeman et al. synthesized a cube made of DNAs (DNA cube) with 10 types of oligonucleotides by a five-step reaction of organization, ligation, purification, reconstruction and ligation (J. Chen, N. C. Seeman, Nature, 350, 631-633 (1991), N. C. Seeman, Acc. Chem. Res., 30, 357-363 (1997)).

However, themethod for synthesis of the DNA cube according to Seeman et al. involves problems that it requires complicate and time-consuming operations and invite high cost. It involves further problems that since the DNA cube is a cube having the size of approximately 10 nm, it is difficult to include nanoparticles such as proteins or the like in the interior and to use that as a transport carrier of nanoparticles.

Meanwhile, with respect to molecular carrier used as transport carrier, especially drug carrier used in drug delivery system, which has targeting property and releases drugs responding to temperature or DNA molecular recognition, liposome made of lipid molecules has been mainly used widely.

However, there is a problem that energy action such as ultrasonic irradiation is required to prepare liposome.

The present inventors have established a technology to construct sugar clusters along one-dimensional DNA assembly by self-organization through double strand formation of conjugate comprising oligonucleotide and galactose with the half-sliding complementary chain (JP-A-2001-247596, K. Matsuura, M. Hibino, Y. Yamada and K. Kobayashi, J. Am. Chem. Soc., 123, 357-358 (2001)).

Thus, development of DNA nanocages which have characters inherent in DNA molecule (namely molecular recognition by base pairing and self-organization by hybridization), can be constructed at one step by self-organization without energy , and can include nanoparticles in the interior, has been highly demanded.

Accordingly, the invention aims to provide a nanocage which is made of DNAs and can be formed by mixing only three types of oligonucleotides without energy, to provide a process for producing DNA nanocages in which the DNA nanocage can be easily and economically constructed by one-step procedure, and to provide DNA nanotubes in which DNA nanocages are linked in a one-dimensional direction, and molecular carrier which can include reversibly many nanoparticles such as metal nanoparticles or proteins in the interior.

### Disclosure of the Invention

The present inventors have assiduously conducted investigations to solve the foregoing problems, and have consequently found that a concept of assembling one-dimensional DNAs by self-organization is extended to two- and three-dimensional concept to obtain spherical DNA nanocages in which metal nanoparticles, proteins or the like can be included reversibly. This finding has led to the completion of the invention.

That is, the invention is specified by the matters described in [1] to [6] below.
[1] DNA nanocage S characterized in that the nanocages are formed by self-organization of tridirectionally branched double-strand DNAs made of three types of sequence-designed oligonucleotides.
[2] The DNA nanocages as recited in [1], characterized in that the nanocages are formed spherically.
[3] A process for producing DNA nanocages, characterized by comprising a step of two-dimensionally assembling three types of oligonucleotides by hybridization to form tridirectionally branched double-strand DNAs having self-complementary chains in the terminals, and a step of three-dimensionally self-organizing the resulting tridirectionally branched double-strand DNAs so as to consume all of the self-complementary terminals.
[4] The process for producing the DNA nanocages as recited in [3], characterized in that the three types of oligonucleotides are mixed at from 0 to 10°C for hybridization.
[5] DNA nanotubes characterized in that the DNA nanocages as recited in [1] or [2] are linked and fused in a one-dimensional direction.
[6] A molecular carrier characterized in that metal nanoparticles or proteins are included in the DNA nanocages as recited in [1] or [2].

### Brief Description of the Drawings

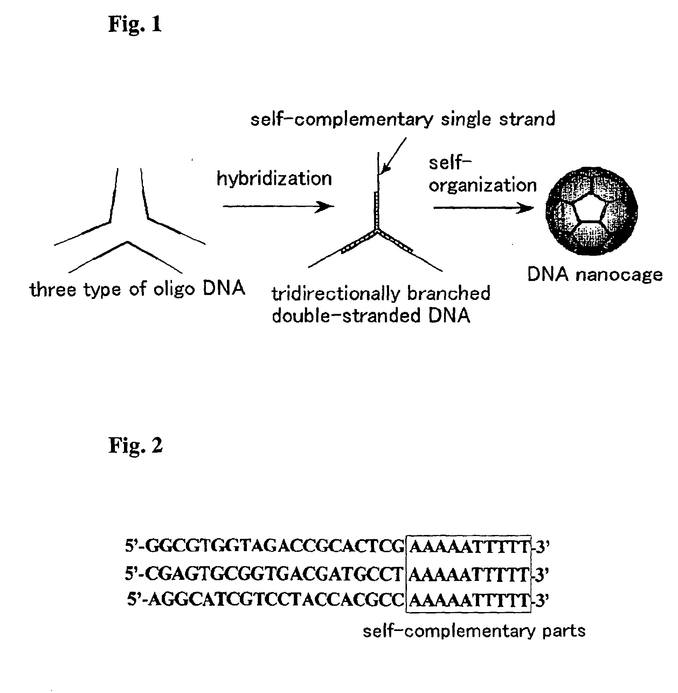

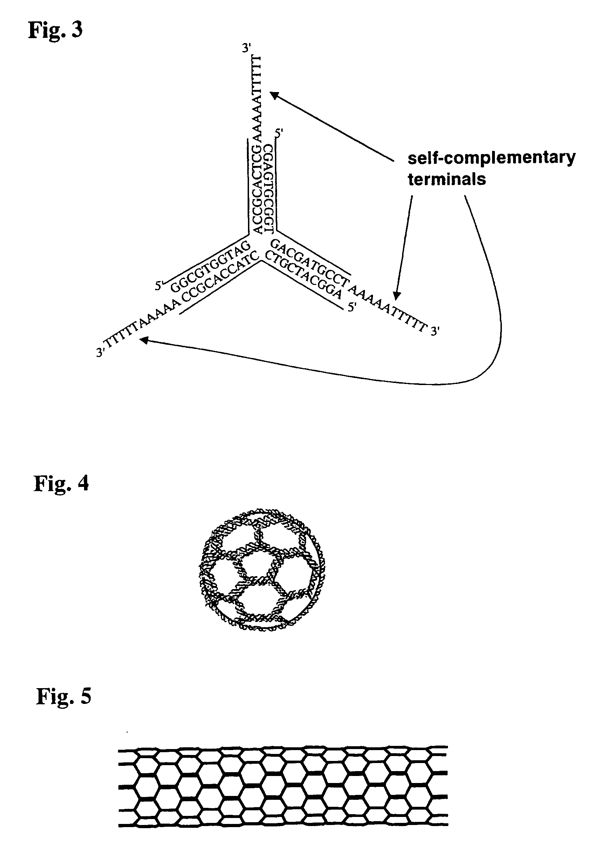

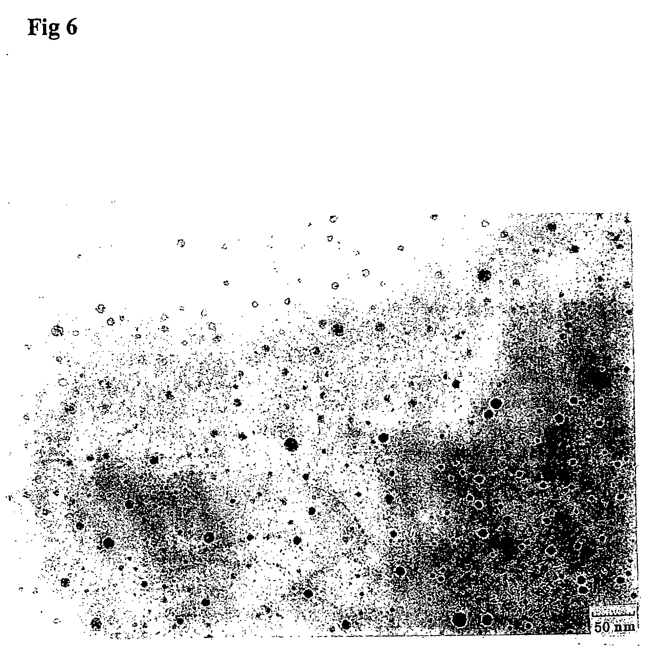

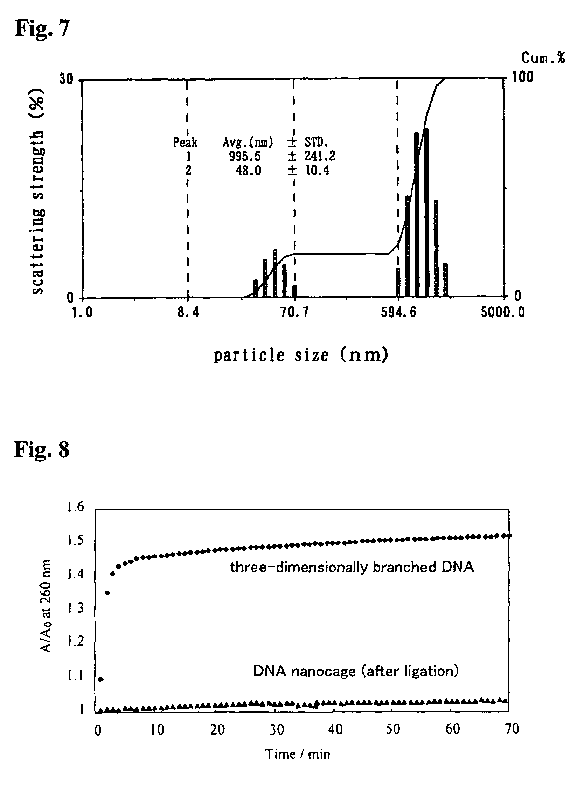

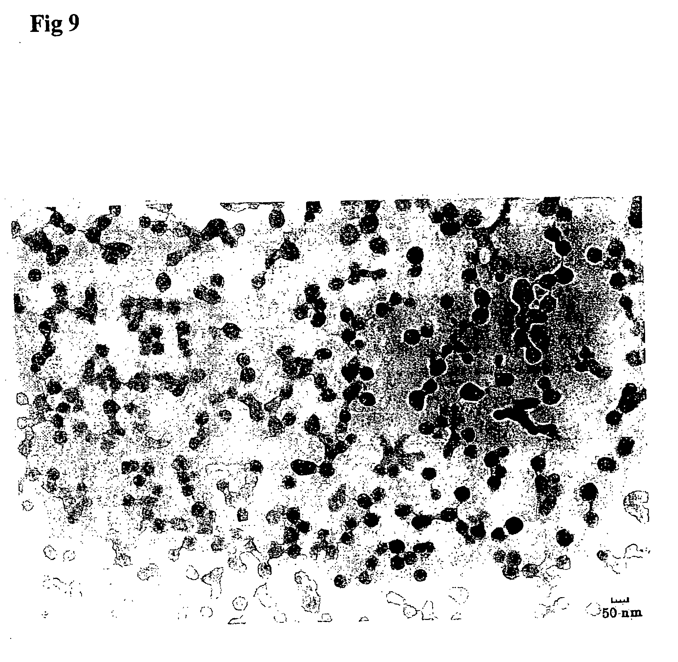

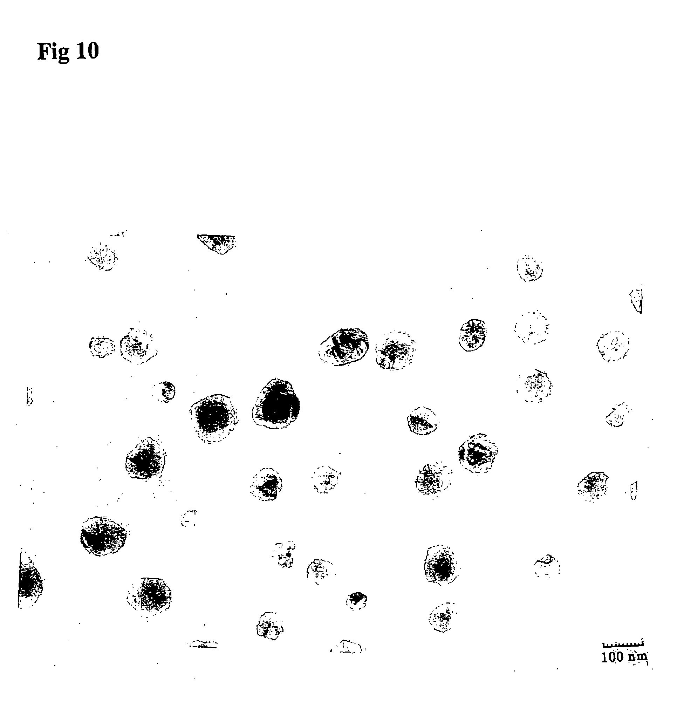

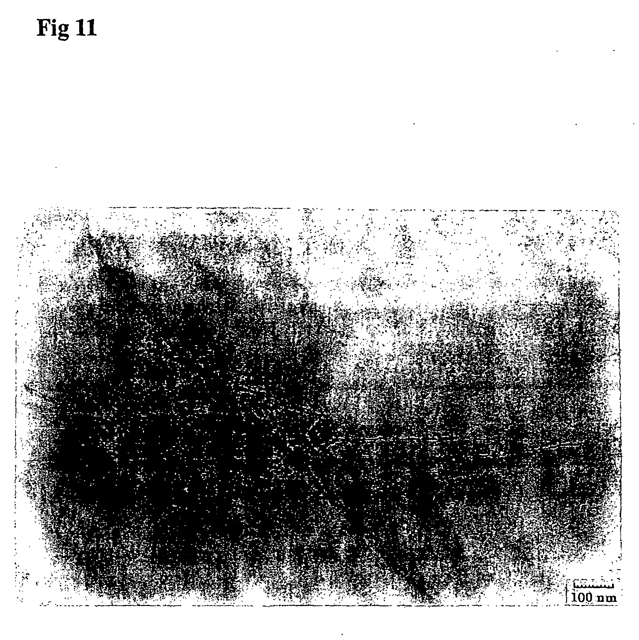

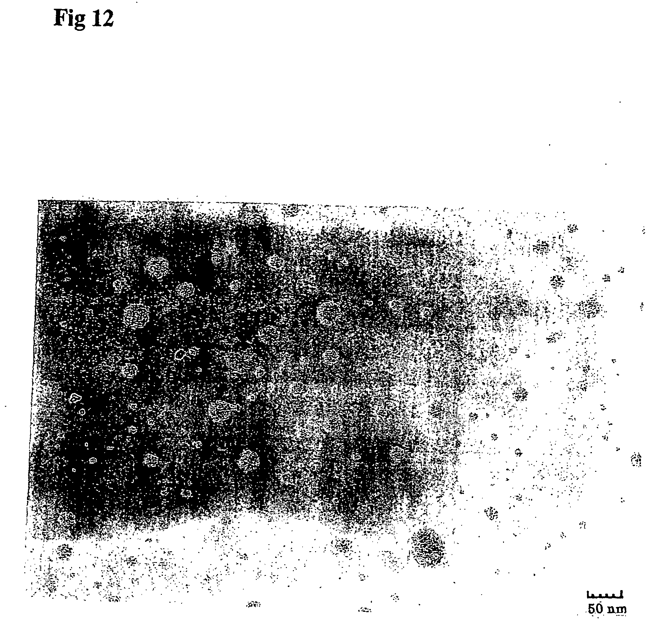

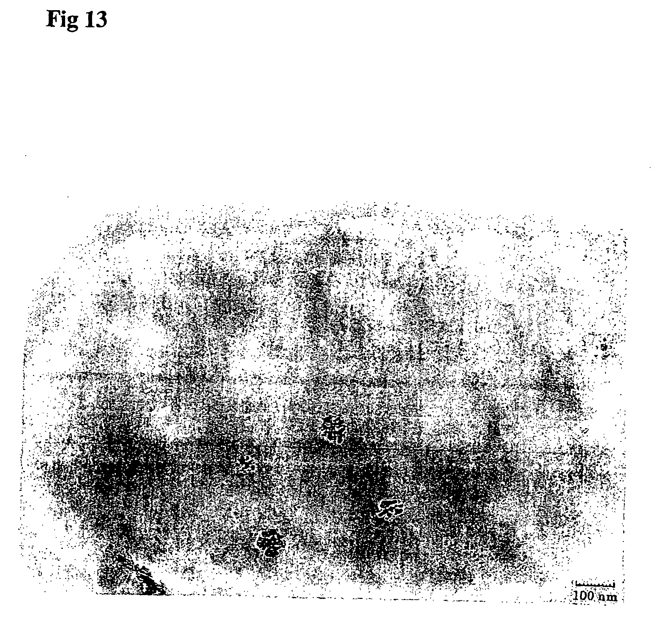

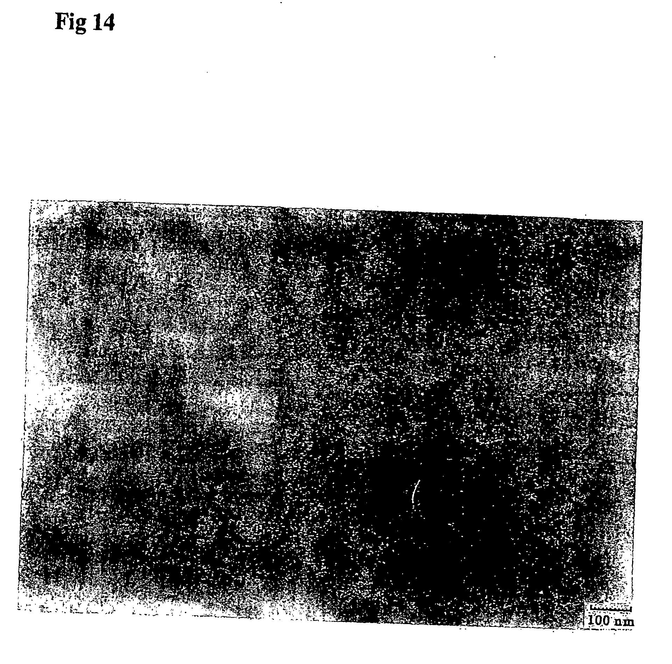

Fig. 1 is a flow chart of producing DNA nanocages in the invention, Fig. 2 is an example of three types of oligonucleotides in the invention, Fig. 3 is an example of a tridirectionally branched double-strand DNA in the invention, Fig. 4 is a model of DNA nanocages in the invention, Fig. 5 is a model of a DNA nanotubes in the invention, Fig. 6 is a transmission electron microscope image of DNA nanocages in Example 1 of the invention, Fig. 7 is a graph showing the particle size distribution by dynamic light scattering of DNA nanocages in Example 1 of the invention, Fig. 8 is a graph showing a time course of the cleavage reaction of DNA nanocages with Exonuclease III, Fig. 9 is a transmission electron microscope image of a network of DNA nanocages in Example 2 of the invention, Fig. 10 is a transmission electron microscope image of DNA nanocages in Example 3 of the invention, Fig. 11 is a transmission electron microscope image of DNA nanotubes in Example 4 of the invention, Fig. 12 is a transmission electron microscope image of cationic gold nanoparticles included in DNA nanocages in Example 5 of the invention, Fig. 13 is a transmission electron microscope image of anionic gold nanoparticles included in DNA nanocages in Example 6 of the invention, and Fig. 14 is a transmission electron microscope image of metalloproteins, ferritins included in DNA nanocages in Example 7 of the invention.

### Best Mode for Carrying Out the Invention

The invention is described in detail below.

A DNA nanocage of the invention can be produced as illustrated in Figure 1, by two-dimensionally assembling three types of oligonucleotides by hybridization to form tridirectionally branched double-strand DNAs having self-complementary chains in the terminals, and three-dimensionally self-organizing said tridirectionally branched double-strand DNAs so as to consume all of the self-complementary terminals.

That is, the DNA nanocages obtained by the process of the invention is so designed that the oligonucleotides are assembled by hybridization to obtain the tridirectionally branched double-strand DNA having the self-complementary chains in the terminals and the resulting double-stranded DNAs are further assembled so as to consume all of the terminals to construct the nanocage.

Accordingly, the DNA sequences are optional so long as they satisfy such conditions of sequence design. For example, sequences of oligonucleotides for producing DNA nanocages are shown in Fig. 2. Three types of oligonucleotide chains are synthesized, which are designed to form a tridirectionally branched double-strand DNA having a self-complementary single-strand DNA in each 3'-terminal. That is, for example, a base sequence is designed which makes it possible to form a trigonal spoke-type complex, a tridirectionally branched double-strand DNA from mainly complementary pairs of guanine and cytosine and further form higher-order structures of DNA using paste sites comprising adenine and thymine.

In the invention, the self-complementary terminal refers to a single-strand DNA terminal having a DNA sequence in which the sequence read from the 5'-end and the sequence read from the 3'-end are complementary (it is said that T is complementary to A and C to G). Such a sequence can form a double-strand DNA by itself. For example, 5'-GCTTCGATCGAAGC-3' is a self-complementary sequence.

In the present specification, the oligonucleotide refers to a compound in which each nucleoside comprising a deoxyribose and a nucleobase is linked through a phosphodiester bond to form oligomer (from several nucleosides to several tens of nucleosides). The double-strand DNA refers to a complex in which oligonucleotides form a double helix by hybridization.

A method for synthesizing three types of oligonucleotide chains in the invention is not limited. Generally, they are synthesized with an automatic synthesizer or the like using a phosphoroamidite method.

It is preferable that the process for producing the DNA nanocages in the invention is performed at a temperature lower than a temperature at which double-strand DNAs are dissociated (melting temperature). The melting temperature depends on the sequences of the oligonucleotides. For example, in the case of oligonucleotides having the lengths and the sequences shown in Fig. 2, the double strand is dissociated into single strands at approximately 35°C.

It is preferable that the three types of oligonucleotides in the invention are mixed at a temperature of from 0 to 10°C for hybridization. Here, when the temperature is outside this range, there is a tendency that the self-complementary terminals are not organized into the cage, though the tridirectionally branched double-strand DNAs are formed.

The length of the three types of oligonucleotides in the invention is preferably from approximately 10-mer to 100-mer. Here, when the length is less than 10-mer, the melting temperature is decreased. For this reason, there is a tendency that the hybridization of the DNA chains is hard to occur and no cage is formed. When the length is more than 100-mer, there is a tendency that any clear structures are hardly observed. The lengths of the three types of oligonucleotides may be the same or different from one another. When the lengths are the same, assemblies with high symmetry are obtained, which is preferable to form spherical cages. It is also possible to construct an asymmetrical structure of, for example, egg shape by using different lengths.

The total concentration of the three types of oligonucleotides in the invention is preferably 1 µM or more. Here, when it is lower than 1 µM, there is a tendency that hybridization does not occur, formation of assemblies is not observed and no DNA nanocages are observed, because there is no chance of sufficiently causing hydrogen bonding of DNAs.

In the process for producing the DNA nanocages according to the invention, salt strength of aqueous solutions of the three types of oligonucleotides is preferably from 0.25 M to 1.0 M. Here, when it is lower than 0.25 M, there is a tendency that the double strands hardly form owing to their electrostatic repulsion and DNA nanocages are hardly observed. When it is higher than 1.0 M, there is a tendency that DNA nanocages are bonded. Accordingly, for observing independent nanocages, the salt strength of approximately 0.5 M is preferable.

In the process for producing the DNA nanocage according to the invention, the salt used is not particularly limited. Monovalent metal salts are available, and the nanocage can be produced regardless of the type of the salt in particular. Of these, NaCl is preferably used.

The DNA nanocages can be obtained by further self-organizing three-dimensionally the tridirectionally branched double-strand DNAs in the invention (refer to Fig. 3) so as to consume all of the self-complementary terminals. The DNA nanocage model is shown in Fig. 4. Their sizes and the shapes can properly be adjusted depending on the DNA sequence, length and concentration of DNA, the salt strength and the like.

In the invention, it is preferable to perform the self-organization of the DNA molecular assemblies in the aqueous solution. It is also possible to perform the self-organization in the gas-liquid interface.

The DNA nanocages according to the invention is cage-shaped assemblies which are made only of DNAs and the inside of which is hollow. Examples of the shape include polygonal structures, spherical and egg shape and the like. The shape is not limited to these shapes, and changed to various types by varying conditions. Especially for facilitating inclusion of nanoparticles in the interior of cages, spherical shape is preferable. Furthermore, it is also possible to form tubular assemblies, DNA nanotubes, by varying the concentrations of the oligonucleotides. For example, when the oligonucleotide concentration is 2 µM and the salt strength is 1 M, spherical assemblies of approximately 50 nm are further organized into the network structure. When the oligonucleotide concentration is 5 µM and the salt strength is 0.5 M, large spherical assemblies of from approximately 50 to 200 nm are formed. When the oligonucleotide concentration is 50 µM or more and the salt strength is 0.5 M, DNA nanotubes with the diameter of approximately 20 nm and the length of 1 µm are formed.

The diameter of DNA nanocages spherically formed in the invention is preferably from 20 to 200 nm. The diameter can properly be changed depending on the purpose of using the DNA nanocages. For example, when the nanocages include 2 or 3 proteins, the cages with small diameter are preferable. When these include large substances such as virus, the cages with large diameter are preferable.

The DNA nanotube according to the invention has a structure that DNA nanocages are linked and fused in a one-dimensional direction. That is, it is considered that the DNA nanocages are melted and grown into a larger molecular assembly, DNA nanotube. The number of the linked DNA nanocages is not particularly limited.

A model of DNA nanotube is shown in Fig. 5.

The size of the DNA nanotubes according to the invention is not particularly limited. It is preferable that the diameter is from approximately 1 to 50 nm and the length is from 100 nm to 10 µm. When the diameter and the length are outside these ranges, the DNA nanotube is deviated from the nano-order region, and is hardly said to be tubular.

The DNA nanocages or the DNA nanotubes according to the invention can include plural nanoparticles in the inner space, examples of the nanoparticles including metal nanoparticles of gold, silver, platinum, palladium and the like, semiconductive nanoparticles of cadmium sulfide, cadmium selenide, zinc sulfide and the like, photocatalytic nanoparticles of titanium oxide and the like, magnetic nanoparticles of iron oxide and the like, silica particles, and nanoparticles of heteropolyacids, plastic fine particles, viruses, proteins, peptides, polysaccharides, another DNA and the like. It can be used as molecular carrier and release the inclusions responding to the environmental change such as temperature or the like.

Specifically, plural cationic and anionic gold nanoparticles or plural protein ferritins can be included in the DNA nanocages, namely cages made of DNAs. In these DNA assemblies, the dissociation and reconstruction can be controlled depending on the temperature or the ionic strength. Consequently, it is considered that a thermo-responsive drug release system can be constructed using the DNA assemblies included protein drugs therein. Since the DNA assemblies can also be dissociated by the presence of the full complementary chain of one DNA chain constituting the DNA assemblies, a drug release system responsive to a specific gene sequence can be constructed. Further, these are useful as a novel drug delivery system by conjugating sugar chains or the like to an oligonucleotide to impart cell-specificity.

Meanwhile, metallic nanoparticles are known to exhibit optical, electrical and magnetic properties different from those of bulk metal owing to quantum size effect.

The DNA nanocages or the DNA nanotubes according to the invention can include plural metal nanoparticles and control the aggregated state of the metal nanoparticles. The nanoparticles are aggregated to develop properties different from those of single nanoparticles. For example, it is clarified that plasmon absorption of gold nanoparticles is shifted to longer wavelength in the formation of the aggregate. In the DNA assembly included the gold nanoparticles therein, the dissociation and the reconstruction can be controlled by temperature or specific molecular recognition, as well as the DNA assembly included proteins therein. Accordingly, a device that changes the optical properties by temperature or specific molecular recognition can be constructed. Moreover, when nanoparticles are prepared in the presence of these DNA assemblies, nanoparticles with specific size and shape can be formed. These methods can also be applied to semiconductive and magnetic particles.

In the invention, the size of the nanoparticles which can be included in the DNA nanocages or the DNA nanotubes is preferably from 2 nm to 200 nm. Here, when the size is smaller than 2 nm, the nanoparticles might leak from space of grids of the DNA nanocages. When the size is larger than 200 nm, the nanoparticles too large to be included in the DNA nanocage.

In the invention, the number of the nanoparticles which can be included in a DNA nanocage or a DNA nanotube can properly be changed depending on the size of the nanoparticles.

In order to facilitate the binding of the nanoparticles to the DNA nanocages in the invention, the nanoparticles can be prepared by using cationic or anionic protective molecules.

The nanoparticles in the invention are different in adhesion site to the DNA nanocages or the DNA nanotubes depending on the surface charge. For example, in case of the positive charge, it is considered that the nanoparticles adhere to (inner and outer) surfaces of the DNA nanocages. In case of the neutral and negative charges, it is considered that the nanoparticles present in the inner aqueous phase of the DNA nanocages and do not directly interact with DNAs.

A method for including the nanoparticles in the DNA nanocages or the DNA nanotubes is not particularly limited. The aqueous solution of the DNA nanocage is once heated to approximately 70°C in the presence of the nanoparticles to dissociate the nanocage, and the temperature is then decreased to 10°C, whereby the nanocage is reconstructed and the nanoparticles are then included therein.

### Examples

The invention is illustrated in more detail below by referring to Examples. However, these Examples are mere working examples, and are not intended to limit the invention. Further, these may be changed without deviating from the scope of the invention.

### Example 1

Three types of 30-mer oligonucleotides with sequences shown in Fig. 2 were mixed in a 0.5 M NaCl aqueous solution at 10°C to be the total oligonucleotide concentration = 1 µM, and the solution was aged for 12 hours.

This solution was dropped on a TEM grid at 10°C, and the specimen was stained with uranyl acetate and observed with a transmission electron microscope. The results are shown in Fig. 6.

In Fig. 6, spherical assemblies with diameters of from approximately 20 to 70 nm were observed.

By dynamic light scanning (DLS) measurement, it was also confirmed that the assemblies with diameters of from 20 to 70 nm were constructed, as shown in Fig. 7. In the Figure, a peak is observed also in the vicinity of 700 nm in addition to from 20 to 70 nm, indicating that the cages are aggregated and the peak apparently appears.

From these experimental results, it is considered that the spherical DNA assemblies observed in Fig. 6 are constructed by hybridizing three types of oligonucleotides to form tridirectionally branched double-strand DNAs having self-complementary chains in the terminals and then assembling the double-stranded DNAs so as to consume all of the self-complementary terminals.

Subsequently, the digestion activity with nucleases which digest DNA from the terminal was measured to confirm the structure of the spherical DNA assemblies.

That is, the spherical DNA assemblies obtained above were ligated with a ligase, and excess single-stranded DNAs were removed with a Mung Bean Nuclease. Subsequently, the digestion activity was measured with Exonuclease III which specifically digest double strands from the 3'-terminal. The results are shown in Fig. 8.

As is clear from Fig. 8, the ligated DNA assemblies were not digested at all under the conditions that double-strand DNAs bearing the terminals are digested (660 mMTris-HCl buffer, 6.6 mM MgCl₂, pH 8, enzyme 70 unit, 37°C). Accordingly, the spherical DNA assemblies obtained in this Example proved to be nanocage-shaped.

### Example 2

Three types of 30-mer oligonucleotides with the same sequences as in Example 1 were mixed in a 1 MNaCl aqueous solution at 10°C to be the total oligonucleotide concentration = 2 µM, and the solution was aged for 12 hours.

This solution was dropped on a TEM grid at 10°C, and the specimen was stained with uranyl acetate and observed with a transmission electron microscope. The results are shown in Fig. 9.

Fig. 9 reveals that the spherical assemblies with diameters of from approximately 30 to 50 nm were further organized into a network structure.

### Example 3

Three types of 30-mer oligonucleotides with the same sequences as in Example 1 were mixed in a 0.5 M NaCl aqueous solution at 10°C to be the total oligonucleotide concentration = 5 µM, and the solution was aged for 12 hours.

This solution was dropped on a TEM grid at 10°C, and the specimen was stained with uranyl acetate and observed with a transmission electron microscope. The results are shown in Fig. 10.

In Fig. 10, the spherical assemblies with diameters of from approximately 50 to 200 nm were observed.

### Example 4

Three types of 30-mer oligonucleotides with the same sequences as in Example 1 were mixed in a 0.5 M NaCl aqueous solution at 10°C to be the total oligonucleotide concentration = 50 µM, and the solution was aged for 12 hours.

This solution was dropped on a TEM grid at 10°C, and the specimen was stained with uranyl acetate and observed with a transmission electron microscope. The results are shown in Fig. 11.

In Fig. 11, it is found that a DNA nanotube with a diameter of approximately 20 nm and a length of 1 µm is formed.

### Comparative Example 1

Three types of 30-mer oligonucleotides with the same sequences as in Example 1 were mixed in a 0.5 M NaCl aqueous solution at 30°C to be the total oligonucleotide concentration = 1 µM, and the solution was aged for 12 hours.

This solution was dropped on a TEM grid at 30°C, and the specimen was stained with uranyl acetate and observed with a transmission electron microscope.

As a result, since the temperature at which to mix the three types of oligonucleotides was 30°C, the DNA double strand of the self-complementary terminal moiety was dissociated, and no spherical assemblies were observed.

### Comparative Example 2

Two types of 30-mer oligonucleotides with the same sequences as in Example 1 were mixed in a 0.5 M NaCl aqueous solution at 10°C to be the total oligonucleotide concentration = 1 µM, and the solution was aged for 12 hours.

This solution was dropped on a TEM grid at 10°C, and the specimen was stained with uranyl acetate and observed with a transmission electron microscope.

As a result, such spherical assemblies as obtained in Example 1 were not observed.

### Comparative Example 3

Three types of 30-mer oligonucleotides which lacks the self-complementary terminals were mixed in a 0.5 M NaCl aqueous solution at 10°C to be the total oligonucleotide concentration = 1 µM, and the solution was aged for 12 hours.

This solution was dropped on a TEM grid at 10°C, and the specimen was stained with uranyl acetate and observed with a transmission electron microscope.

As a result, such spherical assemblies as obtained in Example 1 were not observed.

### Example 5

1 µM of three types of 30-mer oligonucleotides with the same sequences as in Example 1 was organized in a 0.5 mM NaCl aqueous solution at 10°C in the presence of 1.4 mM of cationic gold nanoparticles (average particle diameter 2.2 nm) protected with quaternary ammonium salts to form a molecule carrier.

Subsequently, TEM observation was performed without staining. The results are shown in Fig. 12.

In Fig. 12, it was found that the gold nanoparticles formed aggregates of from approximately 20 to 70 nm. When the DNAs were stained with uranyl acetate, it was found that the location stained agrees with a moiety in which the gold nanoparticles were aggregated.

The plasmon absorption band of the gold nanoparticles was red-shifted by approximately 26 nm through the organization. This is considered to take place because the gold nanoparticles were included in the DNA nanocage.

### Example 6

A molecule carrier was prepared in the same manner as in Example 5 except that anionic gold nanoparticles were used as guest molecules to be included in the interior of the DNA nanocage.

TEM observation was performed as in Example 5. The results are shown in Fig. 13.

In Fig. 13, it was found that the gold nanoparticles formed aggregates of from approximately 2.5 to 30 nm. When the DNAs were stained with uranyl acetate , it was found that the location stained agrees with a moiety in which the gold nanoparticles were aggregated.

### Example 7

A molecule carrier was prepared in the same manner as in Example 5 except that ferritins, metalloproteins were used as guest molecules to be included in the interior of the DNA nanocage.

TEM observation was performed as in Example 5. The results are shown in Fig. 14.

In Fig. 14, it was found that the ferritins formed aggregates of from approximately 8 to 25 nm. When the DNAs were stained with uranyl acetate, it was found that the location stained agrees with a moiety in which the ferritins were aggregated.

### Industrial Applicability

Since the DNA nanocages according to the invention can easily be formed from DNAs by one-step procedure and include nanoparticles in the interior, the DNA nanocages are significantly effective for the development of functional materials using DNAs. Further, the DNA nanocages have a possibility that it can be developed in wide-ranging technological field from nano-region to mesoscopic region. Thus, it can also be used as a multifunctional material in the next generation. Moreover, it can be used as a novel carrier for drug delivery system by including the protein drugs in the interior and binding cell-targeting molecules to the surface, which has targeting property and slowly releases protein drugs responding to temperature or DNA molecular recognition.

Since the DNA nanocage according to the invention can be formed only by mixing with almost no energy required, it has high usefulness as a molecule carrier.

## Claims

1. DNA nanocages **characterized in that** the nanocages are formed by the self-organization of tridirectionally branched double-strand DNAs each made of three types of sequence-designed oligonucleotides.

2. The DNA nanocages according to claim 1, **characterized in that** the nanocages are formed spherically.

3. A process for producing DNA nanocages, **characterized by** comprising a step of two-dimensionally assembling three types of oligonucleotides by hybridization to form a tridirectionally branched double-strand DNA having self-complementary chains in the terminals, and a step of three-dimensionally self-organizing the resulting tridirectionally branched double-strand DNAs so as to consume all of the self-complementary terminals.

4. The process for producing the DNA nanocage according to claim 3, **characterized in that** the three types of oligonucleotides are mixed at from 0 to 10°C for hybridization.

5. DNA nanotubes **characterized in that** the DNA nanocages according to claim 1 or 2 are linked and fused in a one-dimensional direction.

6. A molecular carrier **characterized in that** metal nanoparticles or proteins are included in the DNA nanocages according to claim 1 or 2.
